(19) Europäisches Patentamt
European Patent Office
Office européen des brevets

(11) Numéro de publication : **0 311 521 B1**

# (12) FASCICULE DE BREVET EUROPEEN

(45) Date de publication du fascicule du brevet : **08.04.92 Bulletin 92/15**

(51) Int. Cl.⁵ : **C07D 501/34,** A61K 31/545, C07C 311/00

(21) Numéro de dépôt : **88402527.1**

(22) Date de dépôt : **06.10.88**

(54) **Dérivés des céphalosphorines à pharmacocinétique améliorée, procédé pour leur préparation, compositions pharmaceutiques les contenant et intermédiaire de synthèse.**

(30) Priorité : **08.10.87 FR 8713925**

(43) Date de publication de la demande : **12.04.89 Bulletin 89/15**

(45) Mention de la délivrance du brevet : **08.04.92 Bulletin 92/15**

(84) Etats contractants désignés : **AT BE CH DE ES FR GB GR IT LI LU NL SE**

(56) Documents cités :
**EP-A- 0 127 543**
**EP-A- 0 178 980**
**EP-A- 0 269 512**

(73) Titulaire : **SANOFI**
**40, Avenue George V**
**F-75008 Paris (FR)**

(72) Inventeur : **Labeeuw, Bernard**
**22, Rue Paul Eluard**
**F-34100 Montpellier (FR)**
Inventeur : **Salhi, Ali**
**639, Rue de Valène**
**F-34980 Saint-Gely-Du-Fesc (FR)**

(74) Mandataire : **Gillard, Marie-Louise et al**
**Cabinet Beau de Loménie 55, Rue d'Amsterdam**
**F-75008 Paris (FR)**

EP 0 311 521 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet des dérivés des céphalosporines à pharmacocinétique améliorée, un procédé pour leur préparation et des compositions pharmaceutiques les contenant. Elle concerne également un nouvel intermédiaire de synthèse desdits dérivés de céphalosporines.

Dans la demande de brevet français n° 84 14 878 publiée le 28/03/86 sous le numéro 2 570 702, la Demanderesse a décrit une famille de dérivés de céphalosporines possédant une activité large aussi bien sur les germes Gram négatif que les germes Gram positif.

Parmi ces dérivés figurent notamment des composés substitués en position 3 par un groupe :

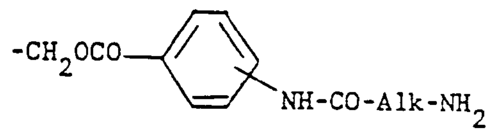

où Alk représente un groupe alkylène inférieur éventuellement substitué et où le substituant NH-CO-Alk-$NH_2$ est situé en position 3 ou 4.

Selon la présente invention, on a trouvé que de façon surprenante en modifiant légèrement la nature du substituant en position 3, on obtenait des composés qui conservaient la bonne activité des composés antérieurement décrits mais présentaient en plus des propriétés pharmacocinétiques très améliorées et notamment des concentrations plasmatiques très élevées persistant pendant un temps très important.

Une telle modification des paramètres pharmacocinétiques est importante dans la mesure où elle permet d'envisager la réduction de la posologie et du nombre de prises du produit pour obtenir un même effet thérapeutique.

Les composés selon l'invention, répondent à la formule générale :

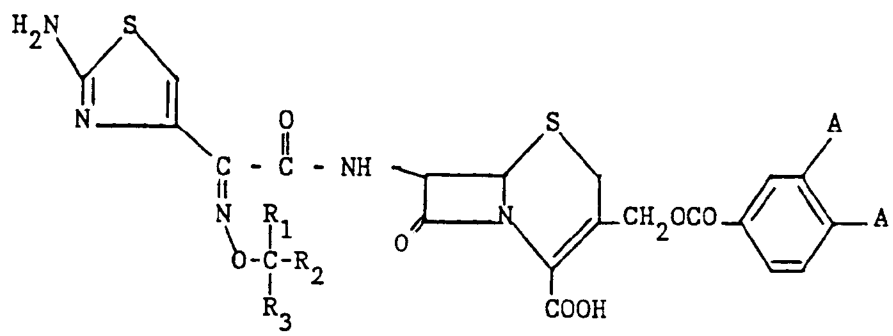 (I)

pdans laquelle :

– $R_1$,$R_2$ et $R_3$ désignent chacun un atome d'hydrogène ou encore $R_1$ et $R_2$ désignent chacun un atome d'hydrogène ou un groupe méthyle et $R_3$ désigne un groupe carboxyle, ou encore $R_1$ et $R_2$ pris ensemble avec l'atome de carbone auquel ils sont liés forment un noyau cyclobutyle et $R_3$ désigne un groupe carboxyle,

– les 2 groupes A sont différents ; l'un représente un groupe OH et l'autre désigne un groupe -$NHSO_2$-Alk-$NH_2$

dans lequel Alk désigne un groupe alkylène inférieur en $C_2$-$C_4$.

Par suite de la présence dans leur formule d'un groupe oxime, les composés (I) peuvent exister sous 2 formes isomères syn et anti. Les isomères syn, dont l'activité thérapeutique est supérieure, sont les composés préférés.

Il est entendu que les composés (I) indiqués ci-dessous peuvent exister

– soit sous la forme indiquée dans la formule (I)

– soit sous la forme tautomère (I') :

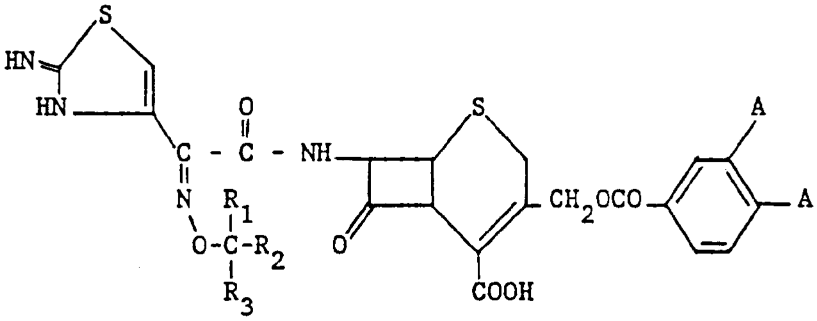 (I')

dans laquelle $R_1$, $R_2$, $R_3$ et A sont tels que définis précédemment.

Les sels des composés de formule I (ou I') font partie intégrante de l'invention.

Il s'agit aussi bien des sels avec les acides pharmaceutiquement acceptables qui peuvent se former avec les fonctions aminées de la molécule que des sels alcalins, alcalinoterreux ou des sels d'aminoacides ou d'amines telles que la triéthylamine ou les éthanolamines qui sont susceptibles de se former avec le groupe carboxyle en position 4 du composé (I) ou lorsqu'il existe avec le groupe carboxyle présent dans le susbstituant de l'oxime ou encore avec les 2 groupes carboxyliques.

Il s'agit également des sels internes, susceptibles de se former entre le (ou les) groupe(s) carboxylique(s) porté(s) par la molécule et les fonctions amine primaires qui y existent sur le substituant A d'une part et le cycle thiazolique d'autre part.

Il en est de même pour les esters facilement hydrolysables ou métaboliquement labiles dérivant de l'un ou de l'autre ou des 2 groupes carboxyliques éventuellement présents dans la molécule.

Parmi ces esters on peut notamment mentionner les esters de phtalidyle :

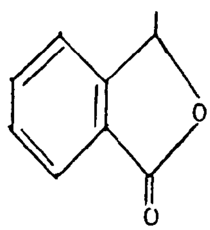

d'acétoxy-1 éthyle 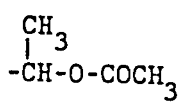

d'éthoxycarbonyloxy-1 éthyle 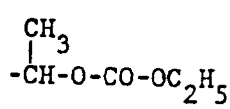

de (méthyl-4 oxo-2 dioxolen-4 yl-5) méthyle 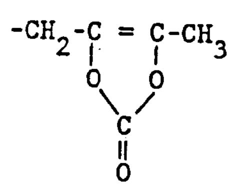

L'invention concerne également un procédé de préparation des composés de formule I (ou I') représenté par le schéma réactionnel suivant :

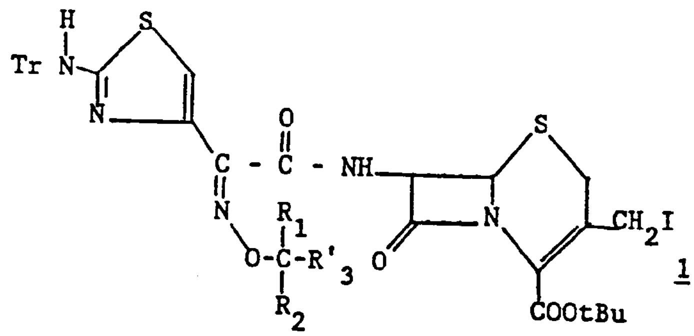

1

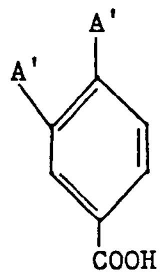

2

--------->

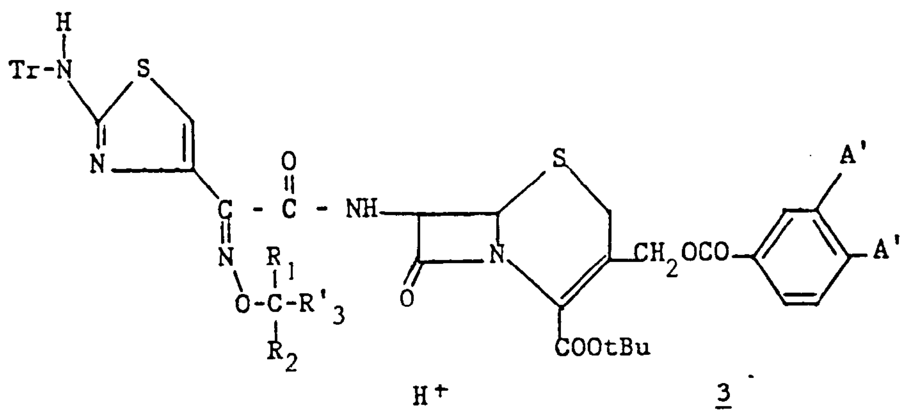

H+ 3

---------> (I)

Dans ces formules, Tr représente un groupe protecteur de la fonction amine, de préférence le group trityle, tBu représente le groupe tertiobutyle et $R'_3$ désigne l'hydrogène ou un groupe ester facilement labile et de préférence un groupe COOtBu.

Les 2 groupes A' sont différents et représentent l'un un groupe OH et l'autre un groupe dérivant du groupe - $NHSO_2AlkNH_2$ par blocage par un groupe labile du groupe aminé qui y est présent.

Enfin $R_1$, $R_2$ et Alk ont les significations précédemment définies.

Sur le composé iodé 1, on fait réagir l'acide 2 (ou l'un de ses dérivés actifs) dans lequel la fonction amine a été préalablement protégée, selon une méthode connue, par un groupement tel que tertiobutoxycarbonyle ou trichloroéthoxycarbonyle.

D'une façon générale la réaction a lieu en solution dans un solvant convenable -aprotique, polaire- de préférence le diméthylformamide en présence de bicarbonate de potassium ou d'une amine tertiaire peu nucléophile comme la diisopropyléthylamine.

La réaction est effectuée à température peu élevée:0 à 20°C.

A partir du composé protégé 3 ainsi obtenu, on prépare les composés (I) par élimination des groupes protecteurs portés par les amines et le ou les esters, selon un procédé connu, en particulier par hydrolyse en milieu acide en utilisant par exemple l'acide trifluoracétique ou un mélange d'acide formique avec un acide fort tel que l'acide chlorhydrique ou l'acide méthanesulfonique.

Dans ces conditions, le composé (I) est isolé directement sous forme de sel du groupement aminé avec l'acide fort utilisé pour la déprotection, c'est à dire sous forme de trifluoracétate, de chlorhydrate, de méthane sulfonate...

Eventuellement, on peut transformer ces sels en d'autres sels d'acide fort, par passage d'une solution de ce sel sur une résine échangeuse d'ions basique sous forme de sel d'acide faible (formiate ou acétate par exemple).

A la solution ainsi obtenue on ajoute l'acide fort dont on veut obtenir le sel et on isole le sel ainsi obtenu par exemple par lyophilisation.

Les sels internes sont préparés par dessalification des sels d'acide fort obtenus lors de la déprotection soit par action d'une base en milieu anhydre, soit par passage sur une colonne de résine échangeuse d'ions.

Les dérivés iodés 1 utilisés comme produits de départ sont connus ou peuvent être préparés selon un procédé connu et notamment comme indiqué dans la demande de brevet allemand n° 3 311 300.

Les amino acides protégés 2 sont préparés à partir des hydroxyamino acides correspondants selon le schéma :

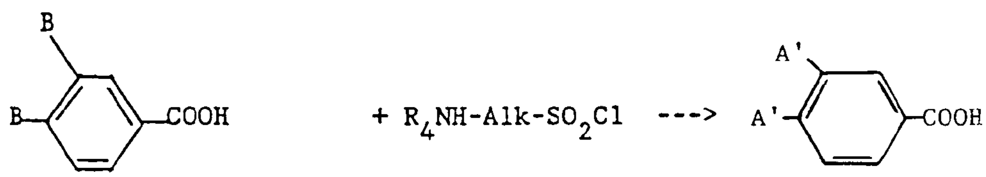


4 5 2

où les groupes B sont différents ; l'un représente OH et l'autre $NH_2$

les groupes A′ sont différents ; l'un représente OH et l'autre un groupe $R_4NH\text{-}Alk\text{-}SO_2$ NH- dans lequel $R_4$ représente un groupe protecteur de la fonction amine, et où Alk a la signification précédemment indiquée.

La réaction est effectuée en solution par exemple dans le chlorure de méthylène en présence d'un accepteur d'acide et d'un réactif qui active la fonction amine et en même temps protège la fonction acide carboxylique. Pour ce faire on utilise de préférence le triméthyl chlorosilane.

A partir des composés 2 où $R_4$ représente un groupe protecteur donné, on peut par hydrolyse acide passer au produit 2 où $R_4$ est l'hydrogène puis selon un procédé connu obtenir un composé 2 où la fonction amine est protégée par un groupe protecteur différent du groupe protecteur initial.

Les composés de formule

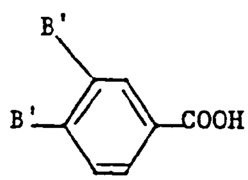

où les 2 groupes B′ sont différents et représentent l'un un groupe OH et l'autre un groupe $X\text{-}NH\text{-}Alk\text{-}SO_2NH\text{-}$ dans lequel X représente l'hydrogène ou un groupe protecteur de la fonction amine et où Alk est tel que défini précédemment sont nouveaux et à ce titre font partie intégrante de l'invention.

Les sels et esters d'acide carboxylique des composés (I) de l'invention s'obtiennent à partir des composés (I) par des réactions connues en elles-mêmes.

Ainsi, les sels minéraux sont obtenus par action sur les composés (I) d'une base minérale telle que la soude ou la potasse ou le bicarbonate de sodium en quantité équimoléculaire ; la réaction de salification est réalisée dans un solvant tel que l'eau ou l'éthanol et le sel obtenu est isolé par évaporation de la solution.

Les sels de bases organiques sont obtenus par action, sur une solution de l'acide I, dans un solvant ou un mélange de solvants convenables, d'une quantité équimoléculaire de la base organique. Le sel est isolé

par précipitation avec l'éther. Les esters sont obtenus par les procédés connus d'estérification ; par exemple, on utilisera avantageusement l'action d'un dérivé halogéné sur un sel tel que le sel de sodium de l'acide ; on réalisera de préférence la réaction dans un solvant capable de dissoudre le dérivé acide de départ, par exemple, dans le diméthylformamide.

Les isomères de forme syn et anti s'obtiennent par un choix convenable des réactifs.

Les exemples suivants permettront de mieux comprendre l'invention.

Ainsi qu'il est habituel dans cette famille de composés, les produits selon l'invention ne présentent pas de point de fusion net, mais seulement des points de décomposition ne permettant pas de les caractériser.

Les produits seront donc caractérisés par leur spectre de résonance magnétique nucléaire. Sauf indication contraire, ils sont enregistrés à 250 MHz, l'étalon interne étant l'hexaméthyldisiloxane.

Les spectres sont enregistrés dans le diméthylsulfoxyde deutérié : 10 mg dans 0,5 ml.

Les déplacements chimiques sont mesurés à ± 0,01 ppm et les constantes de couplage à ± 0,5 Hz.

Les abréviations suivantes seront utilisées :

- S : singulet
- D : doublet
- D de D : double de doublet
- S. e. : singulet élargi
- M : multiplet
- Q : quadruplet
- T : triplet
- AB : système AB
- J : représente la constante de couplage.

De plus, les microanalyses élémentaires ont été effectuées dans chaque cas et sont en accord avec les formules indiquées.

EXEMPLE 1

Bis trifluoracétate de l'acide [(amino-2 thiazolyl-4)-2 méthoxyimino-2 acétamido]-7 [[(amino-2 éthyl sulfonamido)-4 hydroxy-3 benzoyl] oxyméthyl]-3 céphème-3 carboxylique-4 isomère Syn (SR 44337).

$$(I) \quad R_1 = R_2 = R_3 = H \quad A = -OH \quad (3)$$
$$A = -NHSO_2CH_2CH_2NH_2 \quad (4).$$

A) Acide hydroxy-3 [(tertiobutoxycarbonylamino-2 éthyl) sulfonamido ]-4 benzoïque.

1 - Acide hydroxy-3 [(benzyloxycarbonylamino-2 éthyl) sulfonamido]-4 benzoïque.

On met en suspension 84 g d'acide amino-4 hydroxy-3 benzoïque dans 1,8 litres de chlorure de méthylène puis sous atmosphère d'azote, on ajoute 228,5 ml de triéthylamine et refroidit le mélange à 10°C.

On ajoute en 1 heure, sous agitation, 228,5 ml de triméthylchlorosilane.

On laisse remonter la température à 20°C et agite pendant 2 heures 30 à cette température.

On ajoute ensuite à l'abri de la lumière, 168 g de benzyloxycarbonylamino-2 éthane sulfochlorure et agite à température ambiante pendant 6 jours. On verse le mélange réactionnel dans 2 litres de tampon sulfate pH = 2 et 2 litres d'acétate d'éthyle. On sépare la phase organique lave avec de l'eau et sèche sur sulfate de magnésium. On évapore le solvant à siccité et reprend le résidu dans 500 ml d'éther. On essore le solide et rince avec de l'éther isopropylique puis sèche. Poids : 64,5 g.

Par concentration de la solution mère à siccité et traitement du résidu par l'éther isopropylique additionné d'un peu d'éther on isole un second jet du même produit (54 g). On réunit les 2 jets et on dissout dans 470 ml d'éthanol. On ajoute lentement 1,5 litres d'eau et agite à température ambiante pendant 30 minutes puis refroidit la solution dans la glace.

On essore, rince avec de l'eau et sèche sous vide sur anhydride phosphorique. Poids : 102,2g F : 194°C.

2 - Trifluorométhylsulfonate de l'acide hydroxy-3 (amino-2 éthylsulfonamido)-4 benzoïque.

Au mélange refroidi à 10°C de 1 litre d'acide trifluoracétique, 110 ml d'acide trifluorométhane sulfonique et 150 ml de thioanisole, on ajoute sous agitation 102 g du produit obtenu en A).

La température s'élève à 20°C et on agite pendant 1 heure à cette température.

Le produit cristallise et on doit diluer le mélange par 4 litres de chlorure de méthylène. On poursuit l'agitation pendant 15 minutes puis on essore le solide, rince avec du chlorure de méthylène puis sèche sous vide. Poids : 103,8 g. F : 240°C (décomposition).

3- Acide hydroxy-3 [(tertiobutoxycarbonylamino-2 éthyl) sulfonamido]-4 benzoïque.

On dissout 100 g du trifluorométhanesulfonate obtenu ci-dessus dans 1 litre d'eau et on ajuste le pH à 7,5 par addition d'une solution concentrée de soude puis on ajoute 500 ml de dioxanne.

On ajoute en 15 minutes, la solution de 65 g de dicarbonate de ditertiobutyle dans 300 ml de dioxanne tout en maintenant le pH à 7,5 par addition de soude.

Après 30 minutes, le pH reste stable. On lave le mélange réactionnel avec 2,5 litres d'éther puis on verse la phase aqueuse dans 2,5 litres de tampon sulfate pH 2.

On ajuste le pH à 2 par addition de solution de sulfate acide de potassium puis on extrait avec 2 litres d'acétate d'éthyle. On extrait une seconde fois avec 1 litre d'acétate d'éthyle et réunit les extraits organiques. On lave avec 1 litre de solution saturée de chlorure de sodium et sèche la solution sur sulfate de magnésium. On évapore à siccité et reprend le résidu solide dans 1 litre d'acétonitrile. On agite pendant 15 heures à température ambiante puis on refroidit dans la glace et essore le solide. On lave avec de l'éther isopropylique et séché. Poids : 78 g F: 228 - 230°C (décomposition).

B)[(Tritylamino-2 thiazolyl-4)-2 méthoxyimino-2 acétamido]-7 [[(tertiobutoxycarbonylamino-2 éthylsulfonamido)-4 hydroxy-3 benzoyl] oxyméthyl]-3 céphème-3 carboxylate de tertiobutyle isomère syn.

On dissout 37,5 g de l'acide protégé préparé en A) et 15 ml de diisopropyléthylamine dans 200 ml de diméthylformamide. On refroidit le mélange à une température comprise entre 0 et + 5°C, et ajoute 60 g de [(tritylamino-2 thiazolyl-4)-2 méthoxyimino-2 acétamido]-7 iodométhyl-3 céphème-3 carboxylate de tertiobutyle-4 isomère syn.

On laisse sous agitation à 0-5°C pendant 5 heures puis on verse le mélange réactionnel dans 2 litres d'eau glacée. On essore le précipité et rince avec de l'eau. On dissout le solide dans le chlorure de méthylène, sèche la solution sur sulfate de magnésium et évapore le solvant à siccité. On chromatographie sur une colonne de silice H (1kg). En éluant avec le mélange chlorure de méthylène-méthanol 99,1-0,9 vol/vol, on élimine des impuretés puis avec le mélange des mêmes solvants 98-2 vol/vol, on obtient le produit attendu.

Après évaporation des solvants et lavage à l'éther isopropylique, on obtient 30,7 g.

C) SR 44337

Au mélange de 330 ml d'acide trifluoroacétique et 33 ml d'anisole refroidi dans un bain de glace, on ajoute sous agitation 35 g du produit protégé préparé ci-dessus en B).

On laisse remonter la température jusqu'à 20°C et agite pendant 1 heure à cette température.

On verse le mélange dans 1,5 litres d'éther isopropylique refroidi à 0°C.

On essore, lave avec de l'éther puis sèche sous vide sur anhydride phosphorique.

On obtient 26,8 g du produit attendu.

On le dissout dans 100 ml de méthanol et verse la solution dans 1,25 l d'éther. On essore, rince avec de l'éther et sèche sous vide pour obtenir finalement 22 g du produit du titre.

SPECTRE DE RMN

1H à 10,7 ppm (s.e., N<u>H</u>-$SO_2$) - 1H à 9,6 ppm (D,J = 8 Hz, -N<u>H</u>CO-) - 1H à 9,5 ppm (s.e., - O<u>H</u>) - 9H entre 7 et 8,2 ppm (M, 3H aromatiques et 2 N $H_3$) - 1H à 6,7 ppm (s, <u>H</u> thiazole) - 1H à 5,75 ppm (D de D, $J_1$ = 8 Hz, $J_2$ = 4 Hz $H_7$) - 1H à 5,15 ppm (D, J = 4 Hz, $H_6$) - 2 H à 4,9 et 5,2 ppm (AB, $J_{AB}$ = 14 Hz, $C\underline{H_2}OCO$) - 3H à 3,8 ppm (s, $NOC\underline{H_3}$) - 6 H entre 3,1 et 3,75 ppm (M, $C\underline{H_2}NH_2$, $C\underline{H_2}SO_2$, $C\underline{H_2}S$).

EXEMPLE 2

Bis chlorhydrate de l'acide [(amino-2 thiazolyl-4)-2 méthoxyimino-2 acétamido]-7 [[(amino-2 éthylsulfonamido)-4 hydroxy-3 benzoyl] oxyméthyl]-3 céphème-3 carboxylique-4 isomère syn.

On dissout 10,53 g du produit protégé obtenu à l'exemple 1 B dans 60 ml d'acide formique à 98 % et agite la solution pendant 2 heures à 20°C. On ajoute 50 ml d'une solution aqueuse d'acide chlorhydrique 10 N et

agite à nouveau pendant 2 heures à 20°C.

On verse le mélange réactionnel dans 500 ml d'éther éthylique. On essore le bis chlorhydrate, lave avec de l'éther et sèche sous vide.

On obtient 7 g du produit attendu. F : 145°C (décomposition).

SPECTRE DE RMN

1H à 9,8 ppm (D, J = 8 Hz, $N\underline{H}CO$) - 6H à 8,2 ppm (M, 2 $N\underline{H_3}^+$) - 3H entre 7,2 et 7,6 ppm (M, H aromatiques) - 1H à 6,9 ppm (S, H thiazole) - 1H à 5,8 ppm (D de D, $J_1$ = 8 Hz $J_2$ = 4 Hz, $H_7$) - 1H à 5,2 ppm (D, J = 4 Hz, $H_6$) - 2H entre 4,9 et 5,2 ppm (AB, $J_{AB}$ = 14 Hz, $CH_2OCO$) - 3H à 3,9 ppm (S, $-N-OC\underline{H_3}$) - 2H à 3,7 ppm (M, $C\underline{H_2}-S$) - 4H entre 3,2 et 3,5 ppm (M, $-C\underline{H_2}C\underline{H_2}NH_2$).

EXEMPLE 3

Sel interne de l'acide [(amino-2 thiazolyl-4)-2 méthoxyimino-2 acétamido]-7 [[(amino-2 éthylsulfonamido)-4 hydroxy-3 benzoyl] oxy méthyl]-3 céphème-3 carboxylique-4 isomère syn.

On dissout 1 g de bis trifluoroacétate obtenu à l'exemple 1 C) dans 10 ml de diméthylformamide anhydre et on refroidit la solution à 5°C puis on ajoute une solution de 0,24 g de diéthanolamine dans 2 ml de méthanol. On agite le mélange pendant 15 minutes à 5°C puis on le verse dans 100 ml d'éther refroidit à 5°C. On essore le solide et on le sèche sous vide en présence d'anhydride phosphorique.

On obtient 0,800 g de sel interne.

SPECTRE DE RMN

1H à 9,5 ppm (D, J = 8 Hz, $N\underline{H}CO$) - 1H entre 7,5 et 11 ppm (s.e., $O\underline{H}$) - 3H entre 7 et 7,5 ppm (M, H aromatiques) - 1H à 6,7 ppm (S, H thiazole) - 1H à 5,6 ppm (D de D, $J_1$ = 8 Hz, $J_2$ = 4 Hz, $H_7$) - 1H à 5,05 ppm (D, J = 4 Hz, $H_6$) - 2H à 4,85 et 5,15 ppm (AB, $J_{AB}$ = 14 Hz, $C\underline{H_2}OCO$) - 3H à 3,80 ppm (S, $NOC\underline{H_3}$) - 2H à 3,6 ppm (S, $C\underline{H_2}S$) - 2H à 3,12 ppm (M, $C\underline{H_2}SO_2NH$) - 2H à 2,95 ppm (M, $C\underline{H_2}NH_2$).

EXEMPLE 4

Bis trifluoracétate de l'acide [(amino-2 thiazolyl-4)-2 (carboxy-2 propyl-2 oxyimino)-2 acétamido]-7 [[(amino-2 éthylsulfonamido)-4 hydroxy-3 benzoyl] oxyméthyl]-3 céphème-3 carboxylique-4 isomère syn (SR 44338).

(I) $R_1 = R_2 = CH_3$ $R_3 = COOH$ A = OH (3)
A = $-NHSO_2CH_2CH_2NH_2$ (4)

Ce produit est préparé de la même façon que le produit de l'exemple 1, en remplaçant à l'étape B le [(tritylamino-2 thiazolyl-4)-2 méthoxyimino-2 acétamido]-7 iodométhyl-3 céphème-3 carboxylate de tertiobutyle-4 isomère syn par une quantité équivalente de [(tritylamino-2 thiazolyl-4)-2 (tertiobutoxycarbonyl-2 oxyimino)-2 acétamido]-7 iodométhyl-3 céphème-3 carboxylate de tertiobutyle-4 isomère syn.

Après déprotection comme indiqué dans l'exemple 1C, on obtient le produit SR 44338.

SPECTRE DE RMN

1H à 9,4 ppm (D, J = 8 Hz, $CON\underline{H}$) - 11H entre 7 et 11 ppm (M, $\underline{3H}$ aromatiques, $N\underline{H}SO_2$, $O\underline{H}$, $2NH_3$ ) - 1H à 6,7 ppm (s, $\underline{H}$ thiazole) - 1H à 5,8 ppm (D de D, $J_1$ = 8 Hz $J_2$ = 4 Hz, $H_7$) - 1H à 5,2 ppm (D, J = 4 Hz, $H_6$) - 2H à 4,9 et 5,25 ppm (AB, $J_{AB}$ = 14 Hz, $C\underline{H_2}OCO$) - 6H entre 3,1 et 3,75 ppm (M, $C\underline{H_2}NH_2$, $C\underline{H_2}SO_2$, $C\underline{H_2}S$) - 6H à 1,4 et 1,42 ppm

(2s, 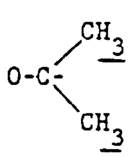 ).

Les produits selon l'invention ont été étudiés en ce qui concerne leurs propriétés pharmacologiques.

L'action bactériostatique a été déterminée in vitro par la méthode des dilutions. L'étude a porté à la fois sur des souches à Gram positif et sur des souches à Gram négatif.

Les résultats, sont exprimés en concentrations minimales inhibitrices (CMI - µg/ml).

A titre d'exemple les résultats obtenus avec SR 44337 (exemple 1) sont réunis dans le tableau 1.

TABLEAU 1

| SOUCHES | SR 44337 |
|---|---|
| Staph. aureus Smith | 0,25 |
| Escherichia coli C1/Col E1 : Tn3 | 0,06 |
| Escherichia coli SOL RL 90 | 0,5 |
| Klebsiella pneumoniae R30 | 4 |
| Proteus vulgaris GN 76/C-1 | 1 |
| Providencia 155 | 1 |
| Pseudomonas aeruginosa NCTC 8203 | 2 |

Ces résultats montrent que les produits selon l'invention présentent un spectre d'activité large et sont dotés d'une très bonne activité intrinsèque.

Par ailleurs le comportement pharmacocinétique des produits selon l'invention a été étudié chez le babouin après administration à la dose de 20 mg/kg par injection intraveineuse.

A partir de prélèvements sanguins effectués à divers temps après l'administration, on détermine la concentration plasmatique du produit étudié par dosage microbiologique.

On peut ainsi construire la courbe donnant la concentration plasmatique en fonction du temps et déterminer différents paramètres pharmacocinétiques du composé étudié :

– la demi vie d' élimination (t1/2 β) est calculée par la formule $1n \frac{2}{\beta}$

où ß représente la pente d'élimination

– l'aire sous la courbe (AUC) est déterminée par la méthode des trapèzes.

Par ailleurs la liaison protéique est obtenue par comparaison de deux gammes étalon, l'une réalisée dans le plasma de babouin et l'autre dans le tampon phosphate (pH7, 0,03M).

Les concentrations plasmatiques obtenues avec le produit de l'exemple 1 sont réunies dans le tableau 2. Elles sont exprimées en µg/ml.

TABLEAU 2

| TEMPS (heures) | µg/ml SR 44337 (Exemple 1) |
|---|---|
| 0,08 | 265,5 |
| 0,16 | 250,9 |
| 0,25 | 237,5 |
| 0,33 | 206,1 |
| 0,5 | 194,3 |
| 0,75 | 190,0 |
| 1 | 182,5 |
| 1,5 | 163,8 |
| 2 | 152,7 |
| 3 | 151,7 |
| 4 | 146,7 |
| 5 | 141,1 |
| 6 | 120,1 |
| 24 | 39,0 |
| 48 | 14,4 |

Dans le tableau 3 ci-après, on a rassemblé pour le produit de l'exemple 1 les paramètres pharmacocinétiques déterminés d'après les mêmes expériences.

TABLEAU 3

| Paramètre | Produit SR 44337 (exemple 1) |
|---|---|
| Concentration plasmatique maximale (µg/ml) | 265,5 |
| Concentration plasmatique (µg/ml) à 24 h | 39,0 |
| à 48 h | 14,4 |
| t1/2 β (h) | 13,9 |
| AUC 0-∞ (µg/ml x h) | 3316 |
| Excrétion urinaire (% dose) à 6 h | 14 |

Les résultats figurant dans les tableaux 2 et 3 montrent que les concentrations plasmatiques du produit de l'invention sont extrêmement élevées et prolongées.

Les composés selon l'invention présentent donc des paramètres pharmacocinétiques très interessants qui peuvent permettre de diminuer de façon sensible la quantité de principe actif à utiliser et le nombre d'administrations journalières nécessaires pour obtenir un effet thérapeutique donné.

Enfin la toxicité des produits selon l'invention est suffisamment faible pour permettre leur utilisation en thérapeutique.

Les produits de l'invention peuvent donc être employés comme antibiotiques en médecine humaine ou vétérinaire. Ils présentent un large spectre et peuvent être utilisés dans toutes les infections bactériennes à germes sensibles.

Les produits peuvent être administrés par voie générale (parentérale, orale, rectale) ou par voie topique.

Les compositions pharmaceutiques sont réalisées à partir des composés (I) sous une forme soluble obtenue par salification d'au moins une des fonctions acides ou des fonctions amines qui y sont présentes.

Les compositions pharmaceutiques contenant à titre d'ingrédient actif l'antibiotique selon l'invention en combinaison avec un véhicule pharmaceutiquement acceptable peuvent être solides ou liquides et se présenter, par exemple, sous forme de préparations injectables, de comprimés, gélules, granulés, pommades, crèmes, gels ou suppositoires. La posologie peut varier dans de larges proportions, en particulier suivant le type et la gravité de l'infection à traiter et suivant le mode d'administration.

Le plus souvent, chez l'adulte, par voie injectable, elle est comprise entre 0,250 g et 4g par jour.

A titre d'exemple de préparation galénique, on peut préparer une solution injectable contenant, pour chaque ampoule :

| | |
|---|---|
| SR 44337 | 1 g |
| Eau pour préparation injectable | 5 ml |
| Carbonate de sodium q.s. pour pH = | 6,3 |

De telles compositions pharmaceutiques constituent un objet de l'invention. Leur procédé de préparation en constitue un autre.

Il consiste à mélanger à des excipients convenables une dose efficace d'un dérivé des céphalosporines de formule (I).

## Revendications

### Revendications pour les Etats contractants suivants : AT, BE, CH, FR, GB, IT, LI, LU, NL, SE

1. Dérivés des céphalosporines répondant à la formule générale :

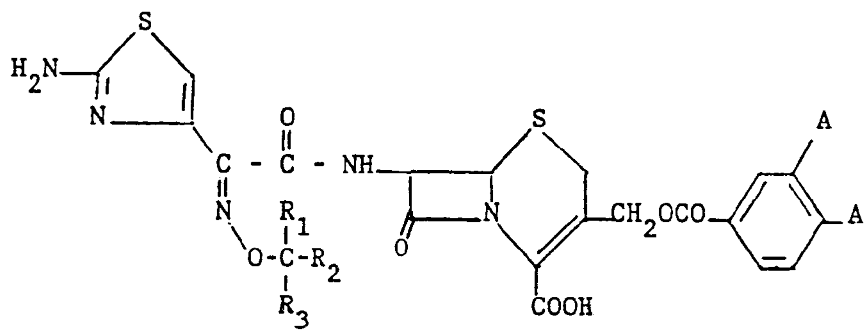

(I)

dans laquelle :

– $R_1$, $R_2$ et $R_3$ désignent chacun un atome d'hydrogène, ou $R_1$ et $R_2$ désignent chacun un atome d'hydrogène ou un groupe méthyle et $R_3$ désigne un groupe carboxyle, ou encore $R_1$ et $R_2$ pris ensemble avec l'atome de carbone auquel ils sont liés forment un noyau cyclobutyle et $R_3$ désigne un groupe carboxyle,

– les 2 groupes A sont différents:l'un représente un groupe hydroxyle et l'autre désigne un groupe $-NH-SO_2-Alk-NH_2$ dans lequel Alk désigne un groupe alkylène inférieur en $C_2$-$C_4$,

ainsi que les sels -y compris les sels internes- et les esters pharmaceutiquement acceptables desdits dérivés.

2. Dérivés des céphalosporines, selon la revendication 1, répondant à la formule générale (I) dans laquelle l'oxime est sous la forme syn.

3. Dérivés des céphalosporines selon la revendication 2, caractérisés en ce qu'ils consistent en l'acide [(amino-2 thiazolyl-4)-2 méthoxyimino-2 acétamido]-7 [[(amino-2 éthyl sulfonamido)-4 hydroxy-3 benzoyl] oxyméthyl]-3 céphème-3 carboxylique-4 ou l'un de ses sels ou esters pharmaceutiquement acceptables.

4. Procédé de préparation des dérivés selon la revendication 1, caractérisé en ce qu'il consiste :

– à faire réagir en solution dans un solvant aprotique polaire sur un composé de formule :

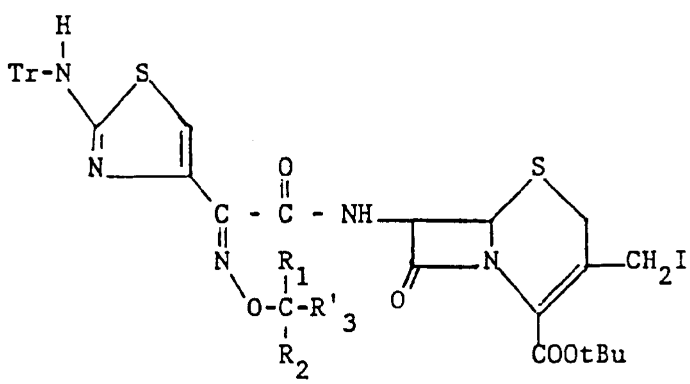

dans laquelle $R_1$ et $R_2$ sont tels que définis à la revendication 1, $R'_3$ désigne l'hydrogène ou un groupe ester facilement labile et Tr représente un groupe protecteur de la fonction amine, un acide de formule

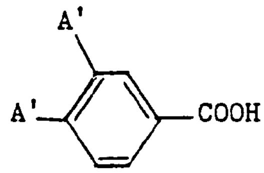

(ou l'un des dérivés réactifs)
dans laquelle les 2 groupes A′ sont différents et représentent l'un un groupe hydroxyle l'autre un groupe correspondant au groupe $-NH-SO_2-Alk-NH_2$ où Alk est tel que défini dans la revendication 1 et dont le groupe aminé a été protégé par un groupe labile,
– à éliminer les groupes protecteurs présents sur les fonctions amines et carboxylique(s) par hydrolyse en milieu acide fort,
– à isoler le composé (I) ainsi obtenu sous la forme d'un sel des groupements aminés présents dans la molécule,
– à transformer éventuellement le dit sel en un sel interne ou en un autre sel des groupements aminés,
– à isoler, éventuellement, à partir d'un sel d'amine le composé I correspondant sous forme de base et à transformer éventuellement celle-ci selon des procédés connus en un sel ou un ester dérivant d'un ou des deux groupes carboxyliques présents dans la molécule.

5. Compositions pharmaceutiques caractérisées en ce qu'elles contiennent en tant que principe actif au moins un composé de formule (I) selon l'une quelconque des revendications 1 à 3.

6. En tant qu'intermédiaires nécessaires à la synthèse des composés de formule (I), les produits nouveaux de formule :

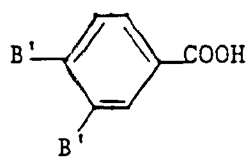

dans laquelle les 2 groupes B′ sont différents et représentent l'un un groupe OH et l'autre un groupe X-NH-Alk-$SO_2$-NH- dans lequel X représente l'hydrogène ou un groupe protecteur de la fonction amine et Alk représente un groupe alkylène inférieur en $C_2$ - $C_4$

**Revendications pour l'Etat contractant suivant : GR**

1. Dérivés des céphalosporines répondant à la formule générale :

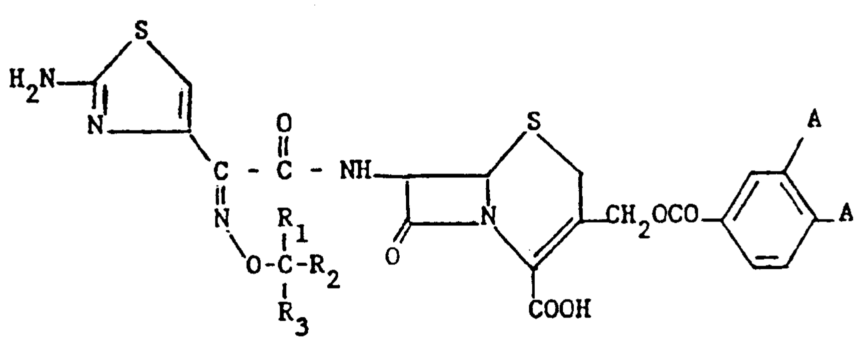

(I)

dans laquelle :
– $R_1$, $R_2$ et $R_3$ désignent chacun un atome d'hydrogène, ou $R_1$ et $R_2$ désignent chacun un atome d'hydrogène ou un groupe méthyle et $R_3$ désigne un groupe carboxyle, ou encore, $R_1$ et $R_2$ pris ensemble avec l'atome de carbone auquel ils sont liés forment un noyau cyclobutyle et $R_3$ désigne un groupe carboxyle,
– les 2 groupes A sont différents : l'un représente un groupe hydroxyle et l'autre désigne un groupe -NH-

$SO_2$-Alk-$NH_2$ dans lequel Alk désigne un groupe alkylène inférieur en $C_2$-$C_4$,
ainsi que les sels -y compris les sels internes- et les esters pharmaceutiquement acceptables desdits dérivés.

2. Dérivés des céphalosporines, selon la revendication 1, répondant à la formule générale (I) dans laquelle l'oxime est sous la forme syn.

3. Dérivés des céphalosporines selon la revendication 2, caractérisés en ce qu'ils consistent en l'acide [(amino-2 thiazolyl-4)-2 méthoxyimino-2 acétamido]-7 [[(amino-2 éthyl sulfonamido)-4 hydroxy-3 benzoyl] oxyméthyl]-3 céphème-3 carboxylique-4 ou l'un de ses sels ou esters pharmaceutiquement acceptables.

4. Procédé de préparation des dérivés selon la revendication 1, caractérisé en ce qu'il consiste :
– à faire réagir en solution dans un solvant aprotique polaire sur un composé de formule :

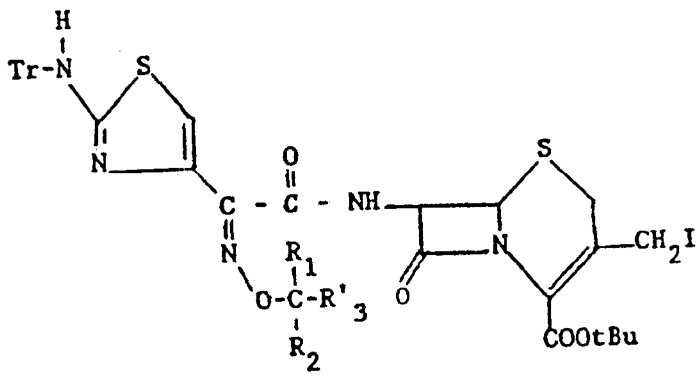

dans laquelle $R_1$ et $R_2$ sont tels que définis à la revendication 1, $R'_3$ désigne l'hydrogène ou un groupe ester facilement labile et Tr représente un groupe protecteur de la fonction amine, un acide de formule :

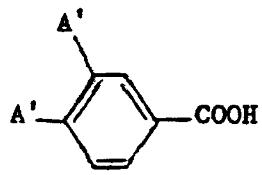

(ou l'un de ses dérivés réactifs)
dans laquelle les 2 groupes A' sont différents et représentent l'un un groupe hydroxyle l'autre un groupe correspondant au groupe -NH-$SO_2$-Alk-$NH_2$ où Alk est tel que défini ci-dessus et dont le groupe aminé a été protégé par un groupe labile,
– à éliminer les groupes protecteurs présents sur les fonctions amines et carboxylique(s) par hydrolyse en milieu acide fort,
– à isoler le composé (I) ainsi obtenu sous la forme d'un sel des groupements aminés présents dans la molécule,
– à transformer éventuellement ledit sel en un sel interne ou en un autre sel des groupements aminés,
– à isoler, éventuellement, à partir d'un sel d'amine le composé I correspondant sous forme de base et à transformer éventuellement celle-ci selon des procédés connus en un sel ou un ester dérivant d'un ou des deux groupes carboxyliques présents dans la molécule.

5. Procédé pour la préparation de compositions pharmaceutiques caractérisé en ce qu'il consiste à mélanger à des excipients convenables une dose efficace d'un dérivé des céphalosporines selon l'une quelconque des revendications 1 à 3.

6. En tant qu'intermédiaires nécessaires à la synthèse des composés de formule (I), les produits nouveaux de formule :

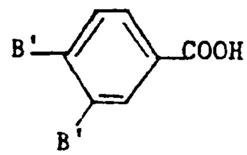

dans laquelle les 2 groupes B′ sont différents et représentent l'un un group OH et l'autre un groupe X-NH-Alk-$SO_2$-NH- dans lequel X représente l'hydrogène ou un groupe protecteur de la fonction amine et Alk représente un groupe alkylène inférieur en $C_2$-$C_4$.

**Revendications pour l'Etat contractant suivant : ES**

1. Procédé pour la préparation de dérivés des céphalosporines répondant à la formule générale :

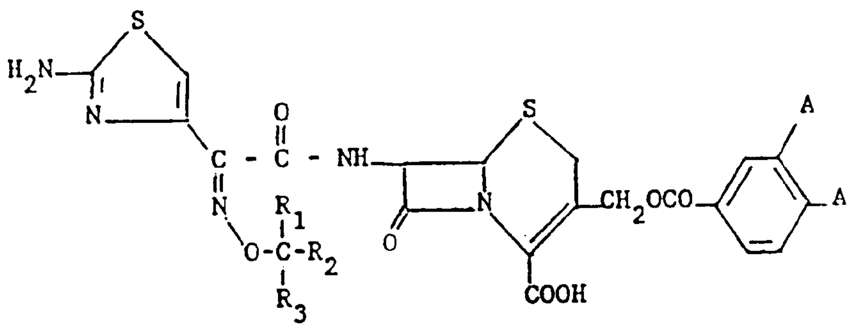

(I)

dans laquelle :

– $R_1$, $R_2$ et $R_3$ désignent chacun un atome d'hydrogène, ou $R_1$ et $R_2$ désignent chacun un atome d'hydrogène ou un groupe méthyle et $R_3$ désigne un groupe carboxyle, ou encore, $R_1$ et $R_2$ pris ensemble avec l'atome de carbone auquel ils sont liés forment un noyau cyclobutyle et $R_3$ désigne un groupe carboxyle,

– les 2 groupes A sont différents : l'un représente un groupe hydroxyle et l'autre désigne un groupe -NH-$SO_2$-Alk-$NH_2$ dans lequel Alk désigne un groupe alkylène inférieur en $C_2$-$C_4$,

ainsi que de leurs sels -y compris leurs sels internes- et de leurs esters pharmaceutiquement acceptables, caractérisé en ce qu'il consiste :

– à faire réagir en solution dans un solvant aprotique polaire sur un composé de formule :

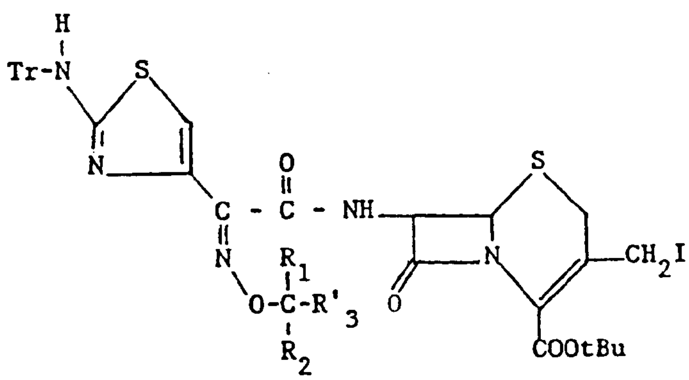

I

dans laquelle $R_1$ et $R_2$ sont tels que définis ci-dessus, $R'_3$ désigne l'hydrogène ou un groupe ester facilement labile et Tr représente un groupe protecteur de la fonction amine, un acide de formule :

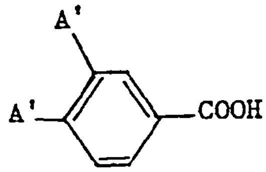 (ou l'un de ses dérivés réactifs) 2

dans laquelle les 2 groupes A′ sont différents et représentent l'un un groupe hydroxyle l'autre un groupe correspondant au groupe $-NH-SO_2-Alk-NH_2$ où Alk est tel que défini ci-dessus et dont le groupe aminé a été protégé par un groupe labile,

– à éliminer les groupes protecteurs présents sur les fonctions amines et carboxylique(s) par hydrolyse en milieu acide fort,

– à isoler le composé (I) ainsi obtenu sous la forme d'un sel des groupements aminés présents dans la molécule,

– à transformer éventuellement ledit sel en un sel interne ou en un autre sel des groupements aminés,

– à isoler, éventuellement, à partir d'un sel d'amine le composé I correspondant sous forme de base et à transformer éventuellement celle-ci selon des procédés connus en un sel ou un ester dérivant d'un ou des deux groupes carboxyliques présents dans la molécule.

2. Procédé selon la revendication 1, caractérisé en ce que le réactif de formule 1 est un isomère syn..

3. Procédé selon la revendication 2, caractérisé en ce qu'il consiste à faire réagir l'acide hydroxy-3 [( benzyloxycarbonylamino-2 éthyl) sulfonamido]-4 benzoïque avec le [(tritylamino-2 thiazolyl-4)-2 acétamido]-7 [[(tertiobutoxycarbonylamino-2 éthylsulfonamido)-4 hydroxy-3 benzoyl] oxyméthyl]-3 céphème-3 carboxylate de tertiobutyle isomère syn,

– à éliminer les groupes protecteurs présents sur les fonctions amines et carboxylique par hydrolyse en milieu acide fort,

– et à isoler le composé (I) ainsi obtenu sous la forme désirée.

4. Procédé pour la préparation de compositions pharmaceutiques caractérisé en ce qu'il consiste à mélanger à des excipients convenables une dose efficace d'un dérivé des céphalosporines de formule (I), préparé selon l'une quelconque des revendications 1 à 3.

5. Procédé selon l'une quelconque des revendications 1 à 3, caractérisé en ce que l'on utilise comme réactif un composé de formule :

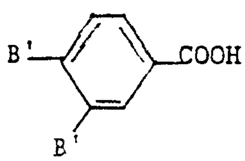

dans laquelle les 2 groupes B′ sont différents et représentent l'un un groupe OH et l'autre un groupe $X-NH-Alk-SO_2-NH-$ dans lequel X représente l'hydrogène ou un groupe protecteur de la fonction amine et Alk représente un groupe alkylène inférieur en $C_2-C_4$.

## Patentansprüche

### Patentansprüche für folgende Vertragsstaaten : AT, BE, CH, DE, FR, GB, IT, LI, LU, NL, SE

1. Cephalosporinderivate der allgemeinen Formel:

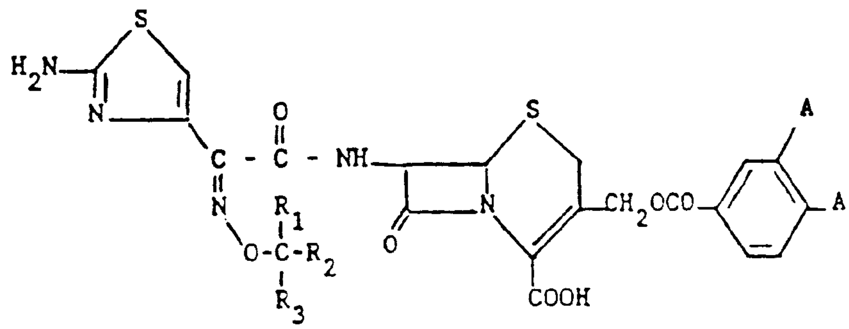

(I)

in welcher:

– $R_1$, $R_2$ und $R_3$ jeweils ein Wasserstoffatom bezeichnen, oder $R_1$ und $R_2$ jeweils ein Wasserstoffatom oder eine Methylgruppe bezeichnen und $R_3$ eine Carboxylgruppe darstellt, oder aber $R_1$ und $R_2$, zusammen mit dem Kohlenstoffatom, an das sie gebünden sind, einen Cyclobutylkern bilden und $R_3$ eine Carboxylgruppe bezeichnet,

– die zwei Gruppen A unterschiedlich sind: die eine eine Hydroxylgruppe und die andere eine Gruppe -NH-$SO_2$-Alk-$NH_2$, in der Alk eine $C_2$-$C_4$-Niedrigalkylengruppe darstellt, bezeichnet,

sowie die pharmazeutisch akzeptablen Salze - einschließlich der internen Salze - und Ester dieser Derivate.

2. Cephalosporinderivate nach Anspruch 1 der allgemeinen Formel (I), in welcher das Oxim in syn-Form vorliegt.

3. Cephalosporinderivate nach Anspruch 2, dadurch gekennzeichnet, daß sie aus der 7-[2-(2-Aminothiazol-4-yl)-2-methoxyimino-acetamido]-3-[[4-(2-aminoethyl-sulfonamido)-3-hydroxy-benzoyl]-oxymethyl]-3-ce phem-4-carbonsäure oder einem ihrer pharmazeutisch akzeptablen Salze oder Ester bestehen.

4. Verfahren zur Herstellung der Derivate nach Anspruch 1, dadurch gekennzeichnet, daß es darin besteht, daß:

– in Lösung in einem polaren aprotischen Lösungsmittel eine Verbindung der Formel

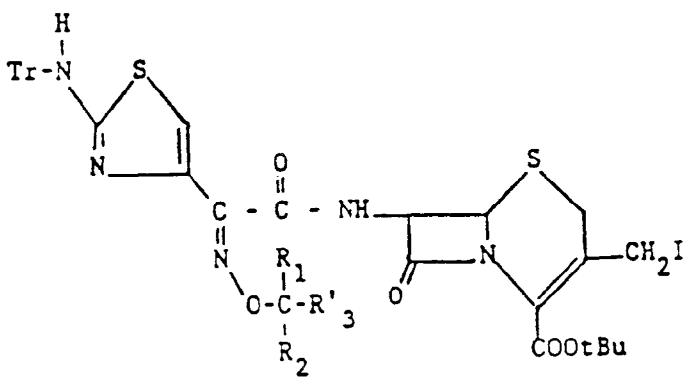

in welcher $R_1$ und $R_2$ wie in Anspruch 1 definiert sind, $R'_3$ Wasserstoff oder eine leicht unbeständige Estergruppe bezeichnet und Tr eine Schutzgruppe der Aminfunktion darstellt, mit einer Säure der Formel

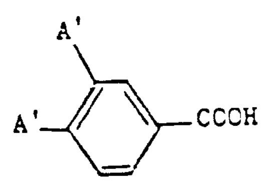

(oder einem ihrer reaktiven Derivate)

in welcher die zwei Gruppen A′ unterschiedlich sind und die eine eine Hydroxylgruppe und die andere eine der Gruppe -NH-$SO_2$-Alk-$NH_2$ entsprechende Gruppe darstellt, worin Alk wie in Anspruch 1 definiert ist,

und deren Aminogruppe durch eine unbeständige Gruppe geschützt ist, reagieren gelassen wird,

– die an den Amin- und Carboxylfunktionen vorhandenen Schutzgruppen durch Hydrolyse in stark saurem Milieu entfernt werden,

– die so erhaltene Verbindung (I) in Form eines Salzes der im Molekül vorhandenen Aminogruppen isoliert wird,

– gegebenenfalls das Salz in ein inneres Salz oder in ein anderes Salz der Aminogruppen übergeführt wird,

– gegebenenfalls die entsprechende Verbindung (I) in Form einer Base aus einem Aminsalz isoliert und gegebenenfalls diese nach bekannten Verfahren in ein Salz oder einen Ester, das bzw. der von einer oder beiden im Molekül vorhandenen Carboxylgruppen stammt, übergeführt wird.

5. Pharmazeutische Zusammensetzungen, dadurch gekennzeichnet, daß sie als Wirkstoff mindestens eine Verbindung der Formel (I) nach einem der Ansprüche 1 bis 3 enthalten.

6. Als für die Synthese der Verbindungen der Formel (I) notwendige Zwischenprodukte die neuen Produkte der Formel

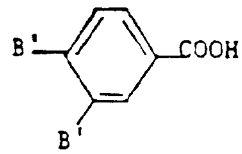

in welcher die zwei Gruppen B′ unterschiedlich sind und die eine eine OH-Gruppe und die andere eine Gruppe X-NH-Alk-$SO_2$-NH-, worin X Wasserstoff oder eine Schutzgruppe der Aminfunktion darstellt und Alk für eine $C_2$-$C_4$-Niedrigalkylengruppe steht, bezeichnen.

**Patentansprüche für folgenden Vertragsstaat: GR**

1. Cephalosporinderivate der allgemeinen Formel:

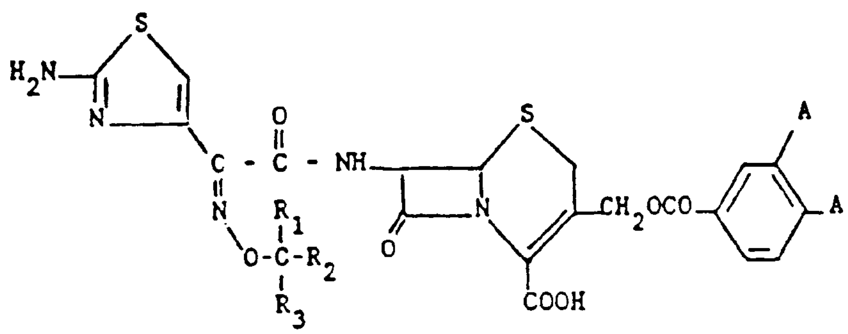

(I)

in welcher:

– $R_1$, $R_2$ und $R_3$ jeweils ein Wasserstoffatom bezeichnen, oder $R_1$ und $R_2$ jeweils ein Wasserstoffatom oder eine Methylgruppe bezeichnen und $R_3$ eine Carboxylgruppe darstellt, oder aber $R_1$ und $R_2$, zusammen mit dem Kohlenstoffatom, an das sie gebunden sind, einen Cyclobutylkern bilden und $R_3$ eine Carboxylgruppe bezeichnet,

– die zwei Gruppen A unterschiedlich sind: die eine eine Hydroxylgruppe und die andere eine Gruppe -NH-$SO_2$-Alk-$NH_2$, in der Alk eine $C_2$-$C_4$-Niedrigalkylengruppe darstellt, bezeichnet,

sowie die pharmazeutisch akzeptablen Salze - einschließlich der internen Salze - und Ester dieser Derivate.

2. Cephalosporinderivate nach Anspruch 1 der allgemeinen Formel (I), in welcher das Oxim in syn-Form vorliegt.

3. Cephalosporinderivate nach Anspruch 2, dadurch gekennzeichnet, daß sie aus der 7-[2-(2-Aminothiazol-4-yl)-2-methoxyimino-acetamido]-3-[[4-(2-aminoethyl-sulfonamido)-3-hydroxy-benzoyl]-oxymethyl]-3-cephem-4-carbonsäure oder einem ihrer pharmazeutisch akzeptablen Salze oder Ester bestehen.

4. Verfahren zur Herstellung der Derivate nach Anspruch 1, dadurch gekennzeichnet, daß es darin besteht, daß:

– in Lösung in einem polaren aprotischen Lösungsmittel eine Verbindung der Formel

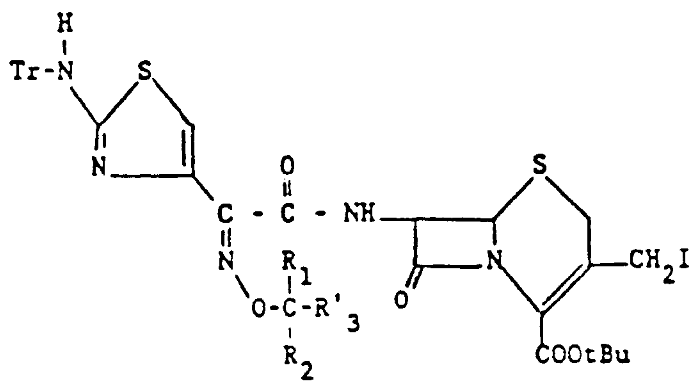

in welcher $R_1$ und $R_2$ wie in Anspruch 1 definiert sind, $R'_3$ Wasserstoff oder eine leicht unbeständige Estergruppe bezeichnet und Tr eine Schutzgruppe der Aminfunktion darstellt, mit einer Säure der Formel

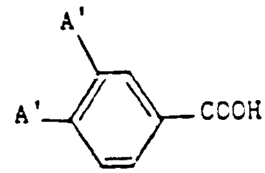

(oder einem ihrer reaktiven Derivate)
in welcher die zwei Gruppen A' unterschiedlich sind und die eine eine Hydroxylgruppe und die andere eine der Gruppe $-NH-SO_2-Alk-NH_2$ entsprechende Gruppe darstellt, worin Alk wie in Anspruch 1 definiert ist, und deren Aminogruppe durch eine unbeständige Gruppe geschützt ist, reagieren gelassen wird,
– die an den Amin- und Carboxylfunktionen vorhandenen Schutzgruppen durch Hydrolyse in stark saurem Milieu entfernt werden,
– die so erhaltene Verbindung (I) in Form eines Salzes der im Molekül vorhandenen Aminogruppen isoliert wird,
– gegebenenfalls das Salz in ein inneres Salz oder in ein anderes Salz der Aminogruppen übergeführt wird,
– gegebenenfalls die entsprechende Verbindung (I) in Form einer Base aus einem Aminsalz isoliert und gegebenenfalls diese nach bekannten Verfahren in ein Salz oder einen Ester, das bzw. der von einer oder beiden im Molekül vorhandenen Carboxylgruppen stammt, übergeführt wird.

5. Verfahren zur Herstellung von pharmazeutischen Zusammensetzungen, dadurch gekennzeichnet, daß es darin besteht, daß geeignete Exzipienten mit einer wirksamen Dosis eines Cephalosporinderivats nach einem der Ansprüche 1 bis 3 vermischt werden.

6. Als für die Synthese der Verbindungen der Formel (I) notwendige Zwischenprodukte die neuen Produkte der Formel

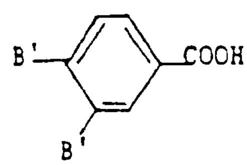

in welcher die zwei Gruppen B' unterschiedlich sind und die eine eine OH-Gruppe und die andere eine Gruppe $X-NH-Alk-SO_2-NH-$, worin X Wasserstoff oder eine Schutzgruppe der Aminfunktion darstellt und Alk für eine $C_2$-$C_4$-Niedrigalkylengruppe steht, bezeichnen.

**Patentansprüche für folgenden Vertragsstaat: ES**

1. Verfahren zur Herstellung von Cephalosporinderivaten der allgemeinen Formel:

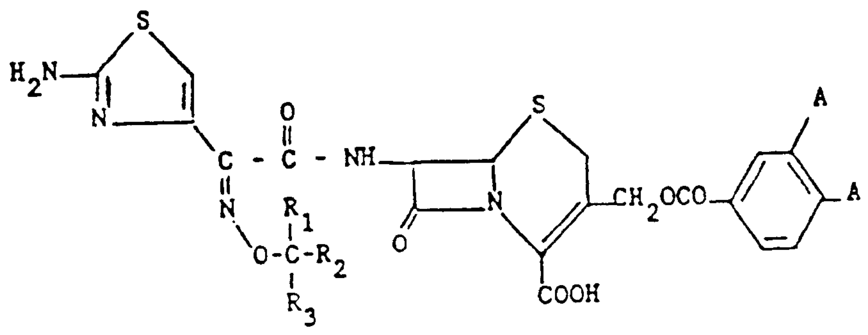

(I)

in welcher:

– $R_1$, $R_2$ und $R_3$ jeweils ein Wasserstoffatom bezeichnen, oder $R_1$ und $R_2$ jeweils ein Wasserstoffatom oder eine Methylgruppe bezeichnen und $R_3$ eine Carboxylgruppe darstellt, oder aber $R_1$ und $R_2$, zusammen mit dem Kohlenstoffatom, an das sie gebunden sind, einen Cyclobutylkern bilden und $R_3$ eine Carboxylgruppe bezeichnet,

– die zwei Gruppen A unterschiedlich sind: die eine eine Hydroxylgruppe und die andere eine Gruppe -NH-$SO_2$-Alk-$NH_2$, in der Alk eine $C_2$-$C_4$-Niedrigalkylengruppe darstellt, bezeichnet,

sowie von deren pharmazeutisch akzeptablen Salzen - einschließlich der internen Salze - und Estern, dadurch gekennzeichnet, daß es darin besteht, daß:

– in Lösung in einem polaren aprotischen Lösungsmittel eine Verbindung der Formel

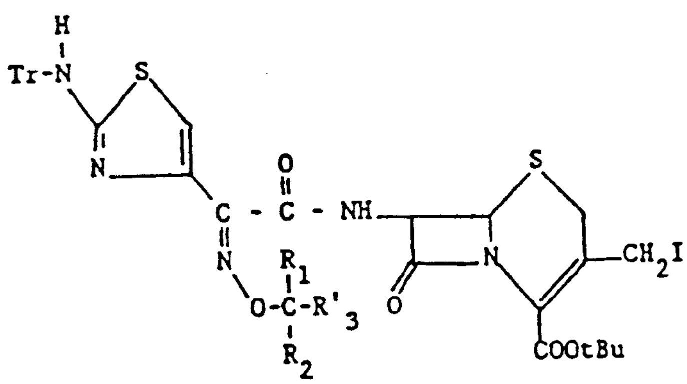

1

in welcher $R_1$ und $R_2$ wie oben definiert sind, $R'_3$ Wasserstoff oder eine leicht unbeständige Estergruppe bezeichnet und Tr eine Schutzgruppe der Aminfunktion darstellt, mit einer Säure der Formel

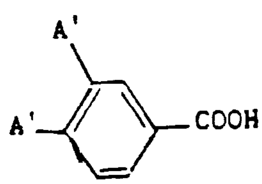

(oder einem ihrer reaktiven Derivate) 2

in welcher die zwei Gruppen A′ unterschiedlich sind und die eine eine Hydroxylgruppe und die andere eine der Gruppe -NH-$SO_2$-Alk-$NH_2$ entsprechende Gruppe darstellt, worin Alk wie oben definiert ist, und deren Aminogruppe durch eine unbeständige Gruppe geschützt ist, reagieren gelassen wird,

– die an den Amin- und Carboxylfunktionen vorhandenen Schutzgruppen durch Hydrolyse in stark saurem Milieu entfernt werden,

– die so erhaltene Verbindung (I) in Form eines Salzes der im Molekül vorhandenen Aminogruppen isoliert wird,

– gegebenenfalls das Salz in ein inneres Salz oder in ein anderes Salz der Aminogruppen übergeführt wird,

– gegebenenfalls die entsprechende Verbindung (I) in Form einer Base aus einem Aminsalz isoliert und gegebenenfalls diese nach bekannten Verfahren in ein Salz oder einen Ester, das bzw. der von einer oder

beiden im Molekül vorhandenen Carboxylgruppen stammt, übergeführt wird.

2. Verfahren nach Anspruch 1, dadurch gekennzeichnet, daß das Reagens der Formel 1 ein syn-Isomeres ist.

3. Verfahren nach Anspruch 2, dadurch gekennzeichnet, daß es darin besteht, daß 3-Hydroxy-4-[(2-benzyloxycarbonyl-aminoethyl)-sulfonamido]-benzoesäure mit 7-[2-(2-Trityl-amino-thiazol-4-yl)-acetamido]-3-[[4-(2-tert.butoxy-carbonylamino-ethylsulfonamido)-3-hydroxybenzoyl]-oxyethyl]-3-cephem-tert.butyl-carboxylat, syn-Isomeres reagieren gelassen wird,

– die an den Amin- und Carboxylfunktionen vorhandenen Schutzgruppen durch Hydrolyse in stark saurem Milieu entfernt werden,

– und die so erhaltene Verbindung (I) in der gewünschten Form isoliert wird.

4. Verfahren zur Herstellung von pharmazeutischen Zusammensetzungen, dadurch gekennzeichnet, daß es darin besteht, daß geeignete Exzipienten mit einer wirksamen Dosis eines Cephalosporinderivats der Formel (I), hergestellt nach einem der Ansprüche 1 bis 3, vermischt werden.

5. Verfahren nach einem der Ansprüche 1 bis 3, dadurch gekennzeichnet, daß man als Reagens eine Verbindung der Formel

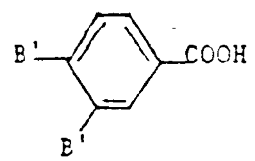

verwendet, in welcher die zwei Gruppen B′ unterschiedlich sind und die eine eine OH-Gruppe und die andere eine Gruppe X-NH-Alk-$SO_2$-NH-, worin X Wasserstoff oder eine Schutzgruppe der Aminfunktion darstellt und Alk für eine $C_2$-$C_4$-Niedrigalkylengruppe steht, bezeichnen.

## Claims

### Claims for the following Contracting State : AT, BE, CH, DE, FR, GB, IT, LI, LU, NL, SE

1. Cephalosporin derivatives of the general formula

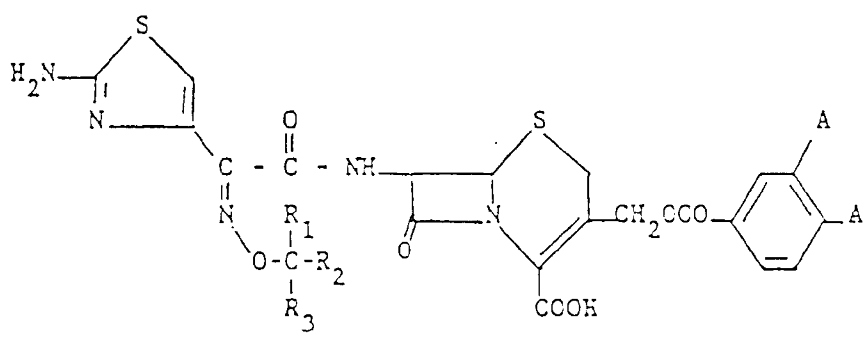

(I)

in which:

– $R_1$, $R_2$ and $R_3$ each denote a hydrogen atom, or $R_1$ and $R_2$ each denote a hydrogen atom or a methyl group and $R_3$ denotes a carboxyl group, or alternatively $R_1$ and $R_2$, taken together with the carbon atom to which they are bonded, form a cyclobutyl ring and $R_3$ denotes a carboxyl group,

– the 2 groups A are different: one representing a hydroxyl group and the other denoting a group NH-$SO_2$-Alk-$NH_2$, in which Alk denotes a $C_2$-$C_4$ lower alkylene group,

as well as the pharmaceutically acceptable salts - including inner salts - and pharmaceutically acceptable esters of the said derivatives.

2. Cephalosporin derivatives according to claim 1 corresponding to general formula (I) in which the oxime is in the syn form.

3. Cephalosporin derivatives according to claim 2, characterized in that they consist of 7-[2-(2-aminothiazol-4-yl)-2-methoxyiminoacetamido]-3-[[4-(2-aminoethylsulfonamido)-3-hydroxybenzoyl]oxymethyl]-3-cephem-4-carboxylic acid or one of its pharmaceutically acceptable salts or esters.

4. A process for the preparation of the derivatives according to claim 1, characterized in that it consists:

– in reacting, in solution in a polar aprotic solvent, a compound of the formula

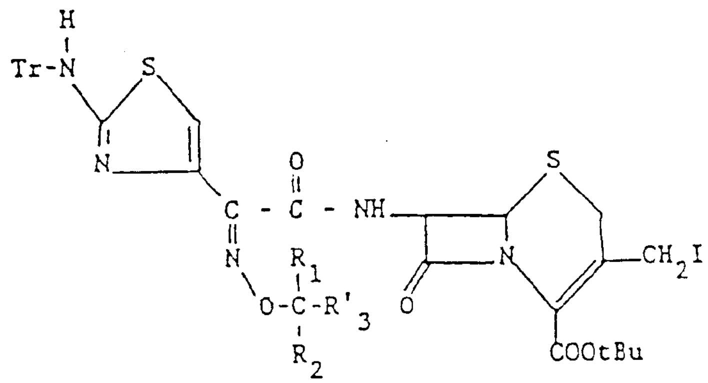

in which $R_1$ and $R_2$ are as defined in claim 1, $R'_3$ denotes hydrogen or a readily labile ester group and Tr represents a protecting group for the amine group, with an acid of the formula

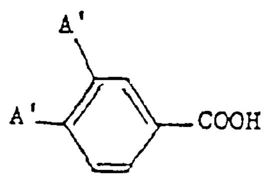

(or one of its reactive derivatives)

in which the 2 groups A′ are different, one of them representing a hydroxyl group and the other representing a group corresponding to the group $-NH-SO_2-Alk-NH_2$, in which Alk is as defined in claim 1 and in which the amino group has been protected by a labile group,

– in removing the protecting groups present on the amine groups and carboxyl group(s) by hydrolysis in a strong acid medium,

– in isolating the resulting compound (I) in the form of a salt of the amino groups present in the molecule,

– in converting, if appropriate, the said salt into an inner salt or another salt of the amino groups, and

– in isolating, if appropriate, from an amine salt, the corresponding compound I in the form of the base and in converting, if appropriate, the latter by known processes into a salt or an ester derived from one or both of the carboxyl groups present in the molecule.

5. Pharmaceutical compositions characterized in that they contain, as the active principle, at least one compound of formula (I) according to any one of claims 1 to 3.

6. As intermediates necessary for synthesizing the compounds of formula (I), the novel products of the formula:

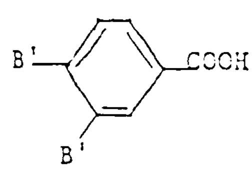

in which the 2 groups B′ are different, one of them representing a group OH and the other representing a group $X-NH-Alk-SO_2-NH-$, in which X represents hydrogen or a protecting group for the amine group and Alk represents a $C_2$-$C_4$ lower alkylene group.

**Claims for the following Contracting State: GR**

1. Cephalosporin derivatives of the general formula

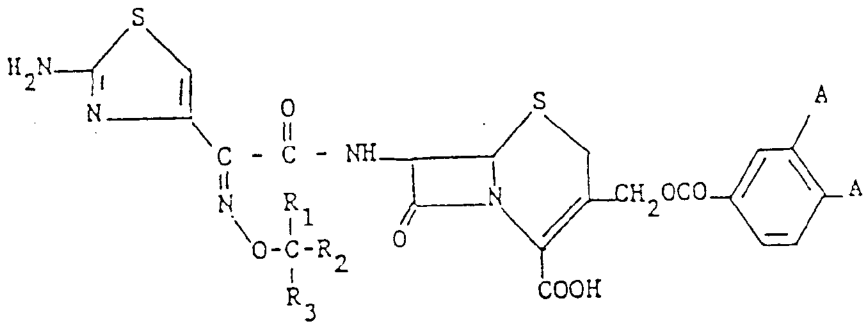

(I)

in which:

– $R_1$, $R_2$ and $R_3$ each denote a hydrogen atom, or $R_1$ and $R_2$ each denote a hydrogen atom or a methyl group and $R_3$ denotes a carboxyl group, or alternatively $R_1$ and $R_2$, taken together with the carbon atom to which they are bonded, form a cyclobutyl ring and $R_3$ denotes a carboxyl group,

– the 2 groups A are different: one representing a hydroxyl group and the other denoting a group $-NH-SO_2-Alk-NH_2$, in which Alk denotes a $C_2$-$C_4$ lower alkylene group,

as well as the pharmaceutically acceptable salts - including inner salts - and pharmaceutically acceptable esters of the said derivatives.

2. Cephalosporin derivatives according to claim 1 corresponding to general formula (I) in which the oxime is in the syn form.

3. Cephalosporin derivatives according to claim 2, characterized in that they consist of 7-[2-(2-aminothiazol-4-yl)-2-methoxyiminoacetamido]-3-[[4-(2-aminoethylsulfonamido)-3-hydroxybenzoyl]oxymethyl-3-cephem-4-carboxylic acid or one of its pharmaceutically acceptable salts or esters.

4. A process for the preparation of the derivatives according to claim 1, characterized in that it consists:

– in reacting, in solution in a polar aprotic solvent, a compound of the formula

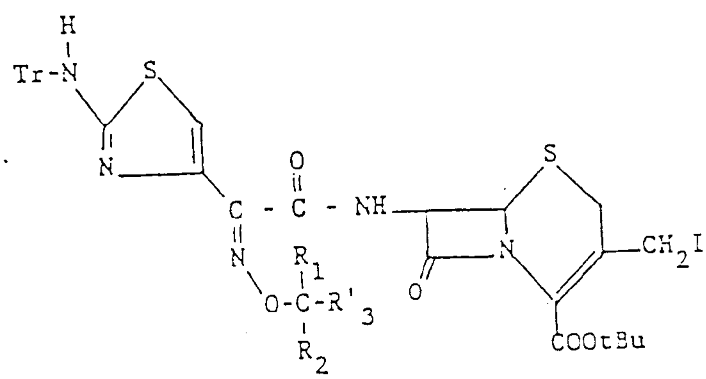

in which $R_1$ and $R_2$ are as defined in claim 1, $R'_3$ denotes hydrogen or a readily labile ester group and Tr represents a protecting group for the amine group, with an acid of the formula

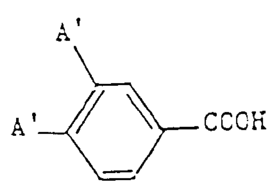

(or one of its reactive derivatives)

in which the 2 groups A' are different, one of them representing a hydroxyl group and the other representing a group corresponding to the group $-NH-SO_2-Alk-NH_2$, in which Alk is as defined in claim 1 and in which the amino group has been protected by a labile group,
– in removing the protecting groups present on the amine groups and carboxyl group(s) by hydrolysis in a strong acid medium,
– in isolating the resulting compound (I) in the form of a salt of the amino groups present in the molecule,
– in converting, if appropriate, the said salt into an inner salt or another salt of the amino groups, and
– in isolating, if appropriate, from an amine salt, the corresponding compound I in the form of the base and in converting, if appropriate, the latter by known processes into a salt or an ester derived from one or both of the carboxyl groups present in the molecule.

5. A process for the preparation of pharmaceutical compositions, characterized in that it consists in mixing an efficient dose of cephalosporin derivatives according to any one of claims 1 to 3, to appropriate excipients.

6. As intermediates necessary for synthesizing the compounds of formula (I), the novel products of the formula:

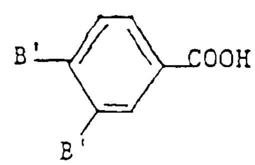

in which the 2 groups B' are different, one of them representing a group OH and the other representing a group $X-NH-Alk-SO_2-NH-$, in which X represents hydrogen or a protecting group for the amine group and Alk represents a $C_2$-$C_4$ lower alkylene group.

**Claims for the following Contracting State: ES**

1. A process for the preparation of cephalosporin derivatives of the general formula

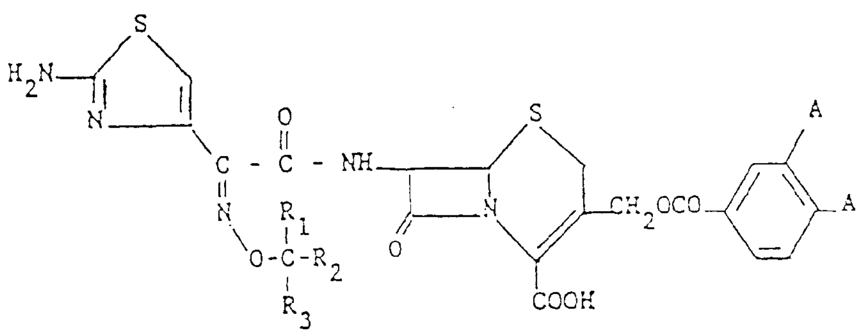

(I)

in which:
– $R_1$, $R_2$ and $R_3$ each denote a hydrogen atom, or $R_1$ and $R_2$ each denote a hydrogen atom or a methyl group and $R_3$ denotes a carboxyl group, or alternatively $R_1$ and $R_2$, taken together with the carbon atom to which they are bonded, form a cyclobutyl ring and $R_3$ denotes a carboxyl group,
– the 2 groups A are different: one representing a hydroxyl group and the other denoting a group $-NH-SO_2-Alk-NH_2$, in which Alk denotes a $C_2$-$C_4$ lower alkylene group,
as well as the pharmaceutically acceptable salts - including inner salts - and pharmaceutically acceptable esters of the said derivatives, characterized in that it consists :
– in reacting, in solution in a polar aprotic solvent, a compound of the formula

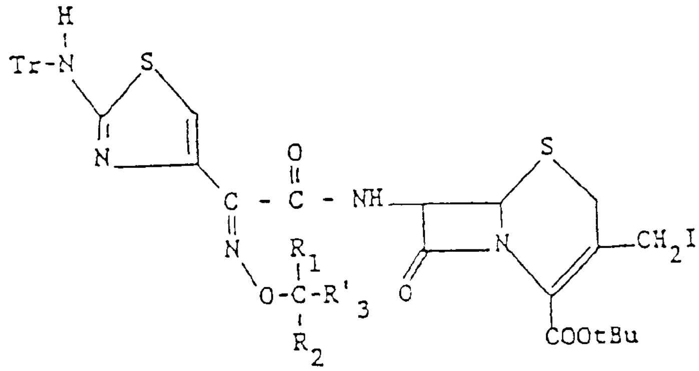

1

in which $R_1$ and $R_2$ are as defined above, $R'_3$ denotes hydrogen or a readily labile ester group and Tr represents a protecting group for the amine group, with an acid of the formula :

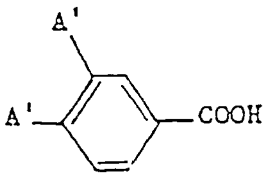

(or one of its reactive derivatives)

2

in which the 2 groups A′ are different, one of them representing a hydroxyl group and the other representing a group corresponding to the group $-NH-SO_2-Alk-NH_2$, in which Alk is as defined above and in which the amino group has been protected by a labile group,

– in removing the protecting groups present on the amine groups and carboxyl group(s) by hydrolysis in a strong acid medium,

– in isolating the resulting compound (I) in the form of a salt of the amino groups present in the molecule,

– in converting, if appropriate, the said salt into an inner salt or another salt of the amino groups, and

– in isolating, if appropriate, from an amine salt, the corresponding compound I in the form of the base and in converting, if appropriate, the latter by known processes into a salt or an ester derived from one or both of the carboxyl groups present in the molecule.

2. The process according to claim 1, characterized in that the reagent of formula 1 is a syn isomer.

3. The process according to claim 2, characterized in that it consists in reacting 3-hydroxy-4-[(2-benzyloxycarbonylaminoethyl)sulfonamido] benzoic acid with 7-[2-(2-tritylaminothiazol-4-yl)-acetamido]-3-[[4-(2-tertiobutoxycarbonylaminoethylsulfonamido)-3-hydroxybenzoyl]-oxymethyl]-3-cephem tertiobutylcarboxylate, syn isomer,

– in removing the protecting groups present on the amine groups and carboxyl group(s) by hydrolysis in a strong acid medium,

– in isolating the resulting compound (I) in the desired form.

4. Process for the preparation of pharmaceutical compositions, characterized in that it consists in mixing an efficient dose of a cephalosporin derivative prepared according to any one of claims 1 to 3, to appropriate excipients.

5. Process according to any one of claims 1 to 3, characterized in that it consists in using as reagent a compound of the formula :

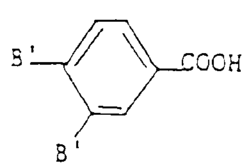

in which the 2 groups B′ are different, one of them representing a group OH and the other representing a group $X-NH-Alk-SO_2-NH-$, in which X represents hydrogen or a protecting group for the amine group and Alk repre-

sents a $C_2$-$C_4$ lower alkylene group.